# EUROPÄISCHE PATENTSCHRIFT

(11) **EP 0 682 022 B1**
(45) Veröffentlichungstag und Bekanntmachung des Hinweises auf die Patenterteilung: **24.07.2002**
(21) Anmeldenummer: 95810287.3
(22) Anmeldetag: 03.05.1995
(51) Int. Cl.: C07D 403/14, C07D 401/14, C07D 405/14, C07D 409/14

(54) **Kondensationsprodukte aus Melamin, (Benzo)triazolen und Aldehyden**
Condensation products of melamine, benzotriazoles and aldehydes
Produits de condensation de mélamine, de benzotriazoles et d'aldéhydes

(30) Priorität: 10.05.1994 CH 145994; 09.11.1994 CH 337494
(43) Veröffentlichungstag der Anmeldung: 15.11.1995
(73) Patentinhaber: Ciba Specialty Chemicals Holding Inc., 4057 Basel (CH)
(72) Erfinder: Wolf, Jean-Pierre, Dr., Ch-1791 Courtaman (DE)

(56) Entgegenhaltungen:
- EP-A- 0 180 992
- DE-A- 1 910 759
- US-A- 4 418 001
- US-A- 4 683 071
- US-A- 4 701 273
- US-A- 4 997 585
- ALAN R. KATRITZKY ET AL.: "The chemistry of n-substituted Benzotriazoles. Part.20." J. CHEM.SOC. PERKINS TRANS. 1,1989, Seiten 639-641, XP002088192 england

## Beschreibung

Die Erfindung betrifft neue, vor allem als Metalldesaktivatoren und Korrosionsinhibitoren geeignete Verbindungen mit Triazin- und Benzotriazolgruppen, diese Verbindungen enthaltende Zusammensetzungen und ihre Verwendung.

Kupferionen katalysieren bekanntlich die Autoxidation sowie die Peroxidradikalbildung in organischen Materialien. Dies trifft auch für den oxidativen Abbau von Schmierstoffen zu. (Vgl. Ullmanns Encyclopedia of Industrial Chemistry, Vol A3, p. 104). Durch Zugabe von Benzotriazol oder Benzotriazol-Derivaten, meist zusammen mit Antioxidantien, kann die Beschleunigung der Schmierstoffzersetzung durch Kupfer drastisch verringert werden.

Man setzt technisch zur Zeit beispielsweise Verbindungen des Typs 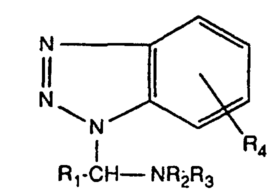 ein, worin z.B. R₁ Wasserstoff, R₂ und R₃ 2-Ethylhexyl oder hydroxyethyl sind und R₄ Wasserstoff oder Methyl ist (s. z.B. US 4,683,071 und US 4,701,273).

Katritzky et al. beschreiben als Zwischenprodukte N-substituierte Benzotriazole der obigen Formel,
in der R₁ Propyl oder Butyl, R₂ und R₄ Wasserstoff und R₃ u.a. Pyridyl sind (A.R. Katritzky, J.-J. Vanden Eynde, J. Chem. Soc. Perkin Trans. **1989**, 639).

In US 4,997,585 wurde z.B. die Verbindung der obigen Formel als Öladditiv beschrieben, in der R₁ n-Heptyl, R₂ Phenyl und R₃ und R₄ Wasserstoff sind.

Es besteht weiterhin ein Bedarf an Wirkstoffen mit metalldesaktivierenden bzw. korrosionsverhindernden Eigenschaften.

Es wurde nun gefunden, daß die im folgenden näher beschriebenen, Triazin- und (Benzo)triazolreste enthaltenden Verbindungen ausgezeichnete metalldesaktivierende und korrosionsverhindernde Eigenschaften aufweisen. Die Wirkung wird weiter verbessert, wenn dem Schmierstoff zusätzlich ein geeignetes Antioxidans oder eine Antioxidansmischung zugesetzt wird.

Die Erfindung betrifft daher Verbindungen der Formel I 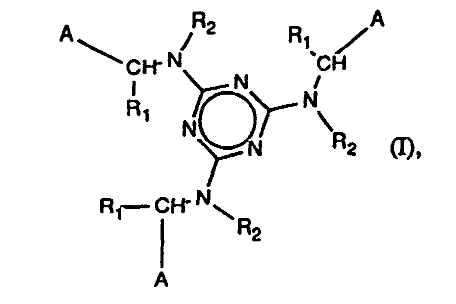 worin
A für 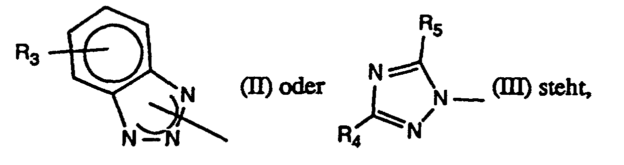 R₁ Wasserstoff, C₁-C₂₀-Alkyl, C₂-C₂₀-Alkenyl, C₅-C₁₂-Cycloalkyl oder mit C₁-C₁₀-Alkyl substituiertes C₅-C₁₂-Cycloalkyl, Phenyl, Naphthyl, mit C₁-C₁₀-Alkyl, C₁-C₁₀-Alkoxy, Halogen, Hydroxy, Nitro oder Phenoxy substituiertes Phenyl oder Naphthyl, eine Gruppe der Formel 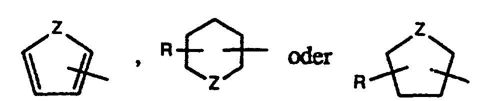 ist, wobei R Wasserstoff oder C₁-C₁₀-Alkyl ist und
Z für Sauerstoff, Schwefel, -NH-, -NR₆- oder eine Methylengruppe steht,
oder R₁ COOR₇ darstellt,
R₂ Wasserstoff, C₁-C₂₀-Alkyl oder C₅-C₁₂-Cycloalkyl ist,
R₃ Wasserstoff, C₁-C₅-Alkyl, C₁-C₅-Alkoxy, Hydroxy, Halogen, Nitro, Carboxy oder C₂-C₁₁-Alkoxycarbonyl ist,
R₄ und R₅ unabhängig voneinander -SH, -NH₂, -NHR₆, -NO₂, -COOH, -SR₆, -N(R₆)₂, -COO-R'₆ oder Maleinimido sind,
R₆ C₁-C₁₀Alkyl bedeutet,
R₇ für Wasserstoff, Mⁿ⁺/n oder [NR₈R₉R₁₀R₁₁]⁺ steht,
M Alkali oder Erdalkalimetall bedeutet, n 1 oder 2 ist, und
R₈ bis R₁₁ unabhängig voneinander Wasserstoff, C₁-C₈-Alkyl oder C₁-C₈-Hydroxyalkyl sind.

Zweckmäßig sind Verbindungen der Formel I, worin
R₁ Wasserstoff, C₁-C₁₈-Alkyl, C₂-C₁₈-Alkenyl, Phenyl, Cyclohexyl, mit C₁-C₄-Alkyl substituiertes Cyclohexyl oder COOR₇ ist,
R₂ Wasserstoff, C₁-C₁₂-Alkyl oder Cyclohexyl ist,
R₃ Wasserstoff, Cl, (C₁-C₅ Alkyl) Methoxy; Hydroxy oder Nitro ist, und R₄ und R₅ unabhängig voneinander für Wasserstoff, SH, NH₂ oder NO₂ stehen.

Bevorzugt sind Verbindungen der Formel I, worin
R₃ Wasserstoff, C₁-C₅-Alkyl, C₁-C₅-Alkoxy oder Hydroxy ist.

Besonders bevorzugt sind Verbindungen der Formel I, worin
R₁ C₁-C₁₂-Alkyl, C₂-C₁₂-Alkenyl, COOR₇ oder Phenyl ist, Verbindungen, worin
R₃ Wasserstoff, Butyl oder Methyl ist,
ferner Verbindungen, worin
R₂ Wasserstoff oder C₁-C₄-Alkyl ist, sowie Verbindungen, worin R₄ und R₅ H sind.
Ebenfalls besonders bevorzugt sind Verbindungen der Formel I, worin
R₂, R₃, R₄ und R₅ Wasserstoff oder C₁-C₄-Alkyl sind und
R₁ C₁-C₁₂-Alkyl, Phenyl, COOH, oder COOR₇ ist.

Eine geeignete Ausführungsform der Erfindung stellen Verbindungen der Formel Ia dar, 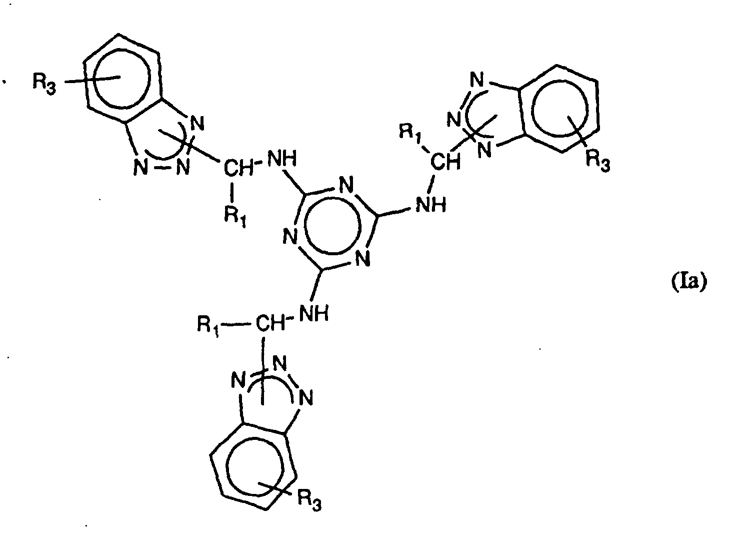 worin R₁ Wasserstoff, C₁-C₂₀-Alkyl, C₂-C₂₀-Alkenyl, C₅-C₁₂-Cycloalkyl, mit C₁-C₁₀-Alkyl substituiertes C₅-C₁₂-Cycloalkyl, Phenyl, Naphthyl, mit C₁-C₁₀-Alkyl, C₁-C₁₀-Alkoxy, Halogen, Hydroxy, Nitro oder Phenoxy substituiertes Phenyl oder Naphthyl, eine Gruppe der Formel 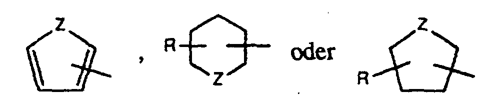 ist, wobei R für Wasserstoff oder C₁-C₁₀-Alkyl steht, Z Sauerstoff oder Schwefel ist, und R₃ Wasserstoff, C₁-C₅-Alkyl, C₁-C₅-Alkoxy, Hydroxy, Halogen, Nitro, Carboxy oder C₂-C₁₁-Alkoxycarbonyl ist, besonders Wasserstoff, C₁-C₅-Alkyl, C₁-C₅-Alkoxy oder Hydroxy ist.
R₁ ist dabei bevorzugt C₁-C₁₂-Alkyl, C₂-C₁₂-Alkenyl oder Phenyl.

Verbindungen der Formel Ia, worin
R₃ Wasserstoff, C₁-C₅-Alkyl, C₁-C₅-Alkoxy oder Hydroxy ist, sowie
Verbindungen der Formel Ia, worin
R₁ C₁-C₁₂-Alkyl, C₂-C₁₂-Alkenyl oder Phenyl ist, sind besonders geeignet.

C₁-C₂₀-Alkyl bedeutende Reste können gerad- oder verzweigtkettig sein und sind je nach der bezeichneten Zahl der C-Atome beispielsweise Methyl, Ethyl, Propyl, Isopropyl, n-Butyl, Isobutyl, t-Butyl, Pentyl, Isopentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl oder Eicosyl.

Bedeuten Reste oder deren Substituenten Gruppen mit einer geringeren Zahl von C-Atomen, so sind entsprechende Beispiele ebenfalls obiger Liste zu entnehmen.

R₃ als Alkylrest hat bevorzugt 1-4 C-Atome und ist insbesondere Methyl.

[NR₈R₉R₁₀R₁₁]^{⊕} bedeutet vorzugsweise Ammonium oder ^{⊕}H₃N-C(CH₃)₂-CH₂-OH.

C₂-C₂₀-Alkenyl kann verzweigt oder unverzweigt sein. Als Beispiele seien erwähnt: Vinyl, Allyl, Methallyl, Hexenyl, Decenyl, Undecenyl, Dimethyl-octadienyl (Geranyl, Noryl), Undecenyl, Heptadecenyl und Oleyl.

C₅-C₁₂-Cycloalkyl bedeutet beispielsweise Cyclopentyl, Cyclohexyl, Cyclooctyl oder Cyclododecyl; bevorzugt sind Cycloalkylreste mit 5-8, insbesondere 5 oder 6 C-Atomen, besonders Cyclohexyl. Beispiele für alkylsubstituierte Cycloalkylgruppen sind Methylcyclohexyl und 4-Butylcyclohexyl.

Halogen ist Chlor, Fluor, Jod oder Brom, insbesondere Chlor.

Durch die Struktureinheit 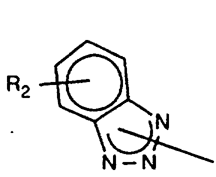 soll in den Formeln I und Ia angedeutet werden, daß jeweils die Substitution in 1- oder 2-Position des Benzotriazols erfolgt sein kann.

Die Herstellung der Verbindungen erfolgt nach in der organischen Chemie geläufigen Methoden (vgl. A.R. Katritzky, J.-J. Vanden Eynde, J. Chem. Soc. Perkin Trans. **1989**, 639), z.B. nach der folgenden Gleichung: 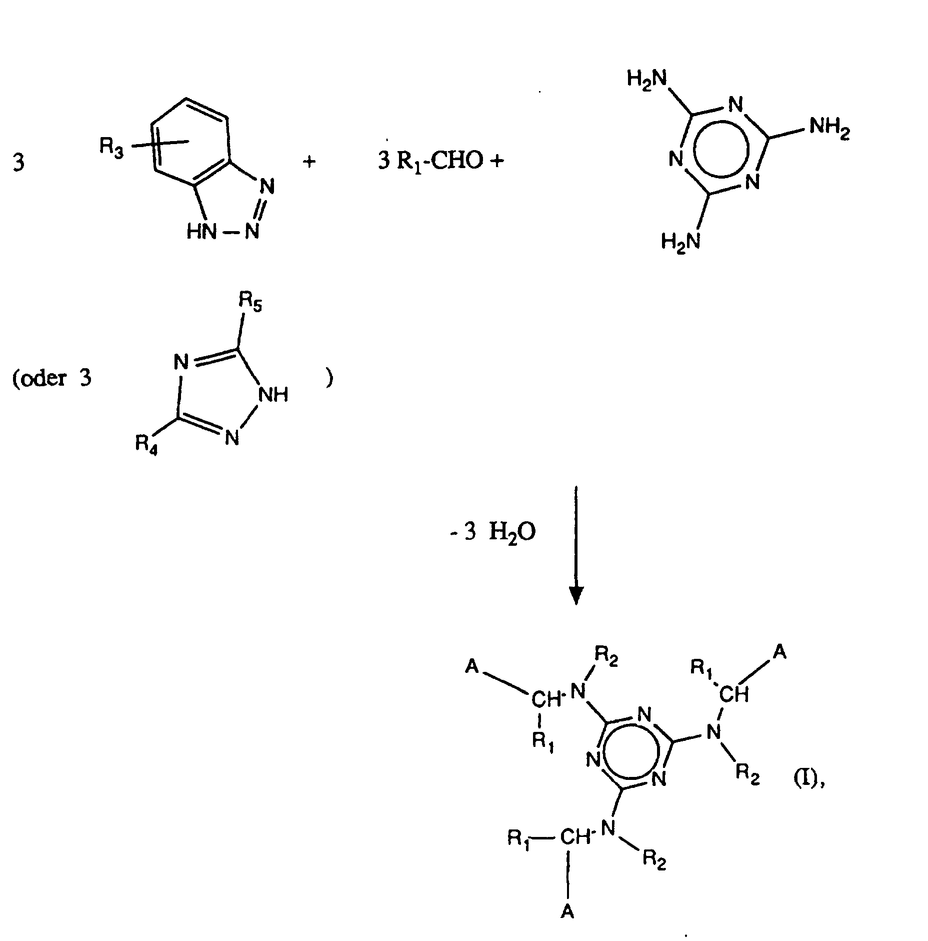 worin
A für

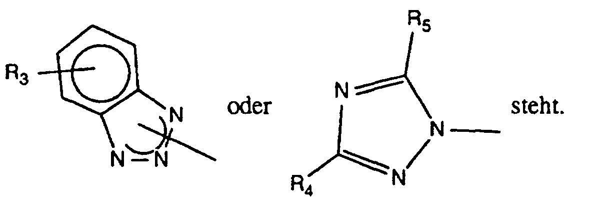

Dabei kann es vorkommen, daß die Umsetzung nicht ganz vollständig ist, so daß nur eine oder 2 (Benzo)triazolgruppen im Molekül enthalten sind. Gegebenenfalls sind diese Nebenprodukte durch geläufige Trennmethoden wie Chromatographie isolierbar. Die Nebenprodukte stören für die bekannten Anwendungen nicht. Diese Nebenprodukte sowie Gemische solcher Nebenprodukte mit den erfindungsgemäßen Verbindungen sind ebenfalls Gegenstand der Erfindung.

Die Kondensation kann in unpolaren organischen Lösungsmitteln unter Katalyse einer Säure wie zweckmäßigerweise para-Toluolsulfonsäure erfolgen.
Die Verbindungen können auch in Alkoholen oder Alkohol/Wasser-Gemischen hergestellt werden, beispielsweise in Ethanol, Methanol oder deren Mischungen mit Wasser. Unter diesen Umständen kann auf die Anwendung eines sauren Katalysators verzichtet werden.

Die Produkte können, wie oben erwähnt, in der 1- oder 2-Position des Benzotriazolsystems substituiert sein (1- oder 2-Benzotriazolylverbindungen). Eine Trennung etwaiger Isomeren ist nicht vonnöten, kann aber mit üblichen Methoden, wie z.B. Chromatographie, erfolgen.

Die eingesetzten Ausgangsstoffe sind kommerziell erhältlich oder nach bekannten Verfahren herstellbar. Es ist darauf hinzuweisen, daß beim Einsatz von Methylbenzotriazol bevorzugt eine Mischung von 4- und 5-Methylbenzotriazol verwendet wird.

Die erfindungsgemäßen Verbindungen eignen sich hervorragend als Metalldesaktivatoren und Antioxidantien für organische Materialien, die in Kontakt mit Metallen kommen oder Metallionen als Verunreinigungen enthalten. Für Schmiermittel zeigt sich auch eine deutliche Verschleißschutzaktivität. Die Erfindung betrifft daher auch Zusammensetzungen enthaltend
a1) einen Schmierstoff, eine Metallbearbeitungs- oder Hydraulikflüssigkeit
   oder
a2) ein Beschichtungs- oder Überzugsmittel, insbesondere einen Lack,
   und
b) mindestens eine Verbindung der Formel I, wobei die oben als bevorzugt genannten Verbindungen zu bevorzugten Zusammensetzungen führen.

Die Verbindungen der Formel I wirken an der Verhinderung von Oxidations- und Zersetzungsprozessen mit, indem sie insbesondere Kupferionen binden und so desaktivieren. Die Erfindung betrifft daher auch die Verwendung von Verbindungen der Formel I als Additive in Schmierstoffen, Hydraulik- und Metallbearbeitungsflüssigkeiten, Beschichtungs- und Überzugsmitteln, insbesondere als Metalldesaktivatoren und Korrosionsinhibitoren.

Die in Frage kommenden Schmierstoffe, Metallbearbeitungs- und Hydraulikflüssigkeiten basieren beispielsweise auf mineralischen oder synthetischen Ölen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Eine weitere Gruppe von Schmierstoffen, die zur Anwendung gelangen können, sind pflanzliche oder tierische Öle, Fette, Talge und Wachse oder deren Gemische untereinander oder Gemische mit den erwähnten mineralischen oder synthetischen Ölen. Pflanzliche und tierische Öle, Fette, Talge und Wachse sind beispielsweise Palmkernöl, Palmöl, Olivenöl, Rüböl, Rapsöl, Leinöl, Erdnußöl, Sojabohnenöl, Baumwollöl, Sonnenblumenöl, Kürbiskernöl, Kokosnußöl, Maisöl, Rizinusöl, Baumnußöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-α-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Caprylund Sebacinsäure oder von einem Gemisch davon. Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Metallbearbeitungsflüssigkeiten und Hydraulikflüssigkeiten können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Erfindungsgemäße Schmierstoffzusammensetzungen finden z.B. Verwendung in Verbrennungsmotoren, z.B. in Kraftfahrzeugen, ausgerüstet z.B. mit Motoren des Otto-, Diesel-, Zweitakt-, Wankel- oder Orbitaltyps.

Die Verbindungen der Formel I sind gut in Schmierstoffen, Metallbearbeitungs- und Hydraulikflüssigkeiten löslich und sind deshalb als Zusätze zu Schmierstoffen, Metallbearbeitungs- und Hydraulikflüssigkeiten besonders geeignet.

Daher betrifft die vorliegende Erfindung ebenfalls ein Verfahren zur Verbesserung der Gebrauchseigenschaften von Schmierstoffen, Metallbearbeitungs- und Hydraulikflüssigkeiten, dadurch gekennzeichnet, daß diesen Verbindungen der Formel I zugesetzt werden.

Die Verbindungen der Formel I können den Schmierstoffen auf an sich bekannte Weise beigemischt werden. Die Verbindungen sind beispielsweise in Ölen gut löslich. Es ist auch möglich, einen sogenannten Masterbatch herzustellen, der mit dem entsprechenden Schmierstoff auf Einsatzkonzentrationen verdünnt werden kann. In solchen Fällen sind auch Konzentrationen über 10 Gew.% möglich.

Die erfindungsgemäßen Verbindungen, wie oben beschrieben, können z.B. in Mengen von 0,01 bis 10 Gew.-%, zweckmäßig in Mengen von 0,05 bis 5 Gew.-%, vorzugsweise in einer Menge von 0,05 bis 3 Gew.-% und ganz besonders bevorzugt von 0,05 bis 1,5 Gew.-%, bezogen auf die Zusammensetzung, in dem Schmierstoff, der Metallbearbeitungs- oder der Hydraulikflüssigkeit vorliegen.

Zusätzlich zu den erfindungsgemäßen Verbindungen können die Schmiermittel, Metallbearbeitungs- und Hydraulikflüssigkeiten noch weitere übliche Additive enthalten, wie z.B. weitere Antioxidantien, Metalldesaktivatoren, Rostinhibitoren, Viskositäts-Index-Verbesserer, Stockpunkterniedriger, Dispergiermittel/Tenside und Hochdruck- und Verschleißschutzadditive (Extreme Pressure/Anti Wear Additives).

### Beispiele hierfür sind:

### Beispiele für phenolische Antioxidantien:

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Ditert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
1.2. Alkylthiomethylphenole, z.B. 2,4-Di-octylthiomethyl-6-tert-butylphenol, 2,4-Dioctylthiomethyl-6-methylphenol, 2,4-Di-octylthiomethyl-6-ethylphenol, 2,6-Di-dodecylthiomethyl-4-nonylphenol.
1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tertbutyl-4-hydroxyanisol, 3,5-Di-tert-butyl-4-hydroxyphenyl-stearat, Bis-(3,5-di-tert-butyl-4-hydroxyphenyl)adipat.
1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylenbis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tertbutyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.
1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat. 1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tertbutyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
1.11. Acylaminophenole, z.B. 4-Hydroxy-laurinsäureanilid, 4-Hydroxystearinsäureanilid, N-(3,5-di-ten-butyl-4-hydroxyphenyl)-carbaminsäureoctylester.
1.12. Ester der β-(3,5-Di-tert-butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.16. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-ditert-butyl-4-hydroxyphenylpropionyl)-trimethylendiamin, N,N'-Bis-(3,5-di-tert-butyl-4-hydroxyphenylpropionyl)-hydrazin.
1.17 Tocopherole, z.B. α-Tocopherol, β-Tocopherol, γ-Tocopherol, δ-Tocopherol und Mischungen davon (Vitamin E).

### Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

### Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,-12-dihydroxy-3,7,10,14-tetrathiahexadecan.

### Beispiele für Metall-Desaktivatoren, z.B. für Kupfer, sind:

a) Benzotriazole und deren Derivate, z.B. 4- oder 5-Alkylbenzotriazole (z.B. Tolyltriazol) und deren Derivate, 4,5,6,7-Tetrahydrobenzotriazol, 5,5'-Methylenbis-benzotriazol; Mannich-Basen von Benzotriazol oder Tolyltriazol wie 1-[Di(2-ethylhexyl)-aminomethyl)-tolyltriazol und 1-[Di(2-ethylhexyl)aminomethyl)-benzotriazol; Alkoxyalkylbenzotriazole wie 1-(Nonyloxymethyl)-benzotriazol, 1-(1-Butoxyethyl)-benzotriazol und 1-(1-Cyclohexyloxybutyl)-tolyltriazol.
b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.
c) Imidazolderivate, z.B. 4,4'-Methylenbis(2-undecyl-5-methylimidazol, Bis[(N-methyl)imidazol-2-yl]carbinol-octylether.
d) Schwefelhaltige heterocyclische Verbindungen, z.B. 2-Mercaptobenzthiazol, 2,5-Dimercapto-1,3,4-thiadiazol und deren Derivate; 3,5-Bis[di(2-ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkylund Alkenylbernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-Carboxymethyl-1-dodecyl-3-methylglycerin und dessen Aminsalze.
b) Stickstoffhaltige Verbindungen, z.B.:
   i. Primäre, sekundäre oder tertiäre aliphatische oder cycloaliphatische Amine und Aminsalze von organischen und anorganischen Säuren, z.B. öllösliche Alkylammoniumcarboxylate, ferner 1-[N,N-bis-(2-hydroxyethyl)amino]-3-(4-nonylphenoxy)-propan-2-ol.
   ii. Heterocyclische Verbindungen, z.B.:
      Substituierte Imidazoline und Oxazoline, 2-Heptadecenyl-1-(2-hydroxyethyl)-imidazolin.
c) Phosphorhaltige Verbindungen, z.B.:
   Aminsalze von Phosphorsäurepartialestern oder Phosphonsäurepartialestern, Zinkdialkyldithiophosphate.
d) Schwefelhaltige Verbindungen, z.B.:
   Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.
e) Glycerinderivate, z.B.:
   Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

### Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

### Beispiele für Stockpunkterniedriger sind:

Polymethacrylat, alkylierte Naphthalinderivate.

### Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

### Beispiele für Hochdruck- und Verschleißschutz-Additive sind:

Schwefel und/oder Phosphor und/oder Halogen enthaltende Verbindungen, wie geschwefelte Olefine und pflanzliche Öle, Zinkdialkyldithiophosphate, alkylierte Triphenylphosphate, Tritolylphosphat, Tricresylphosphat, chlorierte Paraffine, Alkyl- und Aryldi- und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)-thio]-propionsäure-ethylester, Triphenylthiophosphat (Triphenylphosphorothioat), Tris-(alkylphenyl)phosphorothioate und deren Gemische, (z.B. Tris(isononylphenyl)phosphorothioat), Diphenyl-monononylphenyl-phosphorothioat, Isobutylphenyl-diphenylphosphorothioat, Dodecylaminsalz des 3-Hydroxy-1,3-thiaphosphetan-3-oxids, Trithiophosphorsäure-5,5,5-tris[isooctylacetat (2)], Derivate von 2-Mercaptobenzthiazol wie 1-[N,N-Bis(2-ethylhexyl)aminomethyl-2-mercapto-1H-1,3-benzothiazol, Ethoxycarbonyl-5-octyl-dithiocarbamat.

Besonders wirksam sind die erfindungsgemäßen Verbindungen zusammen mit phenolischen und/oder aminischen Antioxidantien.

Überzugsmittel bestehen im allgemeinen aus Bindemitteln, Zusatzstoffen und gegebenenfalls farbgebenden Komponenten.

Als Bindemittel kommen prinzipiell alle in der Technik gebräuchlichen in Betracht, beispielsweise solche, wie sie beschrieben sind in Ullmann's Encyclopedia of Industrial Chemistry, 5. Ed., Vol. A18, pp. 368426, VCH, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Es kann sich um ein kalt aushärtbares oder ein heiß aushärtbares Bindemittel handeln, wobei die Zugabe eines Härtungskatalysators vorteilhaft sein kann. Geeignete Katalysatoren, die die Aushärtung des Bindemittels beschleunigen, sind beispielsweise beschrieben in Ullmann's Encyclopedia of Industrial Chemistry, Vol. A 18, S. 469, VCH Verlagsgesellschaft, Weinheim 1991.
Bevorzugt sind Überzugsmittel, die als filmbildendes Bindemittel Epoxidharze, Polyurethanharze, Polyesterharze, Acrylharze und deren Copolymerharze, Polyvinylharze, Phenolharze, Alkydharze oder Mischungen solcher Harze enthalten.
Die Verbindungen der Formel I können in den Überzugsmitteln zu 0,001 bis 10, bevorzugt zu 0,1 bis 5 % enthalten sein.

Handelt es sich bei den erfindungsgemäßen Zusammensetzungen um Überzugsmittel bzw. Lacke, so können diese ebenfalls weitere übliche Komponenten enthalten, z.B. aus der Gruppe der Farbstoffe, Pigmente, Füllstoffe, Fließkontrollmittel, Haftverbesserer. Härtungskatalysatoren, Lichtschutzmittel oder Antioxidantien.

Bevorzugte erfindungsgemäße Verbindungen, wie vorstehend beschrieben, führen zu bevorzugten Zusammensetzungen.

Die folgenden Beispiele erläutern die Erfindung weiter, ohne sie jedoch zu beschränken. Teile- und Prozentangaben beziehen sich, sofern nicht anders angegeben, auf das Gewicht. Es wird jeweils unter Stickstoff gearbeitet. Wenn die Produkte bereits beim Abkühlen der Reaktionslösung zu kristallisieren beginnen, wird abfiltriert und auf weitere Reinigung verzichtet.

### Beispiele 1 bis 16:

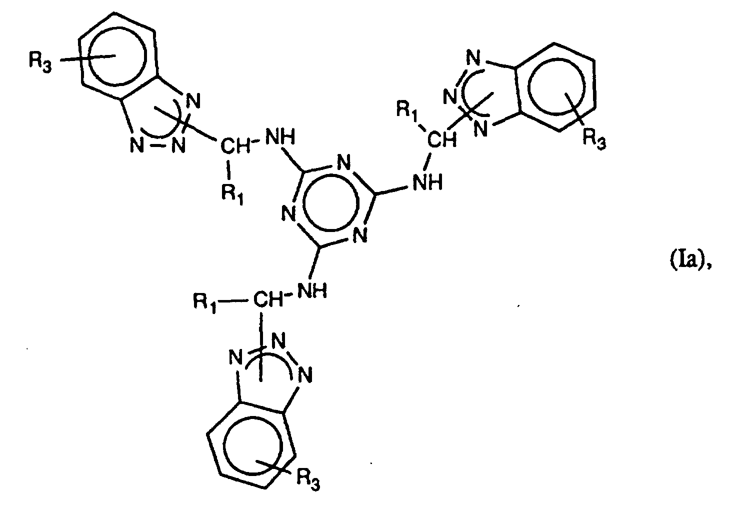

Beispiel 1: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 19,97 g (0,15 mol) Tolyltriazol, 8,5 ml (0,15 mol) Acetaldehyd und 0,2 g para-Toluolsulfonsäure in 100 ml Cyclohexan suspendiert. Die Mischung wird 5 Stunden unter Rückfluß gekocht und währenddessen das entstehende Wasser azeotrop abdestilliert. Das Produkt wird abfiltriert und bei 80°C im Vakuum getrocknet. Man erhält 20,2 g eines orangefarbenen Feststoffes:
Produkt der Formel Ia (R₁= CH₃; R₃ = CH₃)

Beispiel 2: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 19,97 g (0,15 mol) Tolyltriazol und 10,5 ml (0,15 mol) Butyraldehyd und 0,2 g para-Toluolsulfonsäure in 100 ml Cyclohexan suspendiert. Die Mischung wird 12 Stunden unter Rückfluß gekocht und währenddessen das entstehenden Wasser azeotrop abdestilliert. Die Reaktionslösung wird mit 300 ml Toluol versetzt und dreimal mit je 100 ml 5%iger Na₂CO₃-Lösung sowie zweimal mit je 100 ml Wasser gewaschen, über wasserfreiem MgSO₄ getrocknet, und das Lösungsmittel wird abdestilliert. Man erhält 21,4 g eines orangefarbenen Feststoffes: Produkt der Formel Ia (R₁ = CH₃(CH₂)₂, R₃ = CH₃)

Beispiel 2 a: In einem 4-Hals-Kolben mit mechanischem Rührer, Rückflußkühler, Thermometer und Stickstoffeinleitung werden unter Stickstoff 6,31 g (0,05 mol) Melamin, 19,97 g (0,15 mol) Tolyltriazol und 13,5 ml (0,15 mol) Butyraldehyd in 300 ml Methanol suspendiert. Die Suspension wird 14 Stunden unter Rückfluß gekocht und das Lösungsmittel abgezogen. Man erhält in quantitativer Ausbeute das in Beipiel 2 beschriebene Produkt: orangefarbener Feststoff, Produkt der Formel Ia (R₁ = CH₃(CH₂)₂, R₃ = CH₃)

Beispiele 3, 4 und 5: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 19,97 g (0,15 mol) Tolyltriazol und 0,15 mol des betreffenden Aldehyds (vgl. Tabelle I) und 0,2 g para-Toluolsulfonsäure in 100 ml Toluol suspendiert. Die Mischung wird 5 oder 12 Stunden (s. Tabelle I) unter Rückfluß gekocht, und währenddessen wird das entstehende Wasser azeotrop abdestilliert. Die Reaktionslösung wird dreimal mit je 100 ml 5%iger Na₂CO₃-Lösung sowie zweimal mit je 100 ml Wasser gewaschen, über wasserfreiem MgSO₄ getrocknet, und das Lösungsmittel wird abdestilliert. Man erhält jeweils gelbliche Feststoffe.

Beispiel 3: Produkt der Formel Ia (R₁ = CH₃(CH₂)₅, R₃ = CH₃), Smp. 108-26°C

Beispiel 4: Produkt der Formel Ia (R₁ = CH₃(CH₂)₁₀, R₃ = CH₃), Smp. 58-75°C

Beispiel 5: Produkt der Formel Ia (R₁ = C₆H₅, R₃ = CH₃), Smp. 122-138°C

Beispiel 6: In einem 4-Hals-Kolben mit mechanischem Rührer, Rückflußkühler, Thermometer und Stickstoffeinleitung werden unter Stickstoff 12,62 g (0,1 mol) Melamin, 27.62 g (0,3 mol) Glyoxylsäure Monohydrat und 30,95 g (0,3 mol) Tolyltriazol in 150 ml Ethanol und 150 ml Wasser suspendiert. Die Mischung wird 5 Stunden unter Rückfluß gekocht und auf Raumtemperatur abgekühlt. Das Produkt wird abfiltriert, mit 200 ml Wasser gewaschen und im Vakuumtrockenschrank bei 70°C getrocknet. Man erhält 10.3 g (15% d.Th.) eines weißlichen Feststoffes.
Produkt der Formel Ia (R₃ = CH₃, R₁ = CO₂H)

Beispiel 7 (Aminsalz): 3.46 g (0,005 mol) der Verbindung aus Beispiel 6 und 1,5 ml (0.015 mol) 2-Amino-2-methyl-1-propanol werden in 17.3 ml Wasser gelöst und eine Stunde bei Raumtemperatur gerührt. Das Wasser wird am Rotationsverdampfer abdestilliert, und man erhält 4.56 g (95% d.Th.) eines gelblichen, glasigen Feststoffes. Produkt der Formel Ia (R₃ = CH₃, R₁ = CO₂^{⊖ ⊕}H₃N-C(CH₃)₂-CH₂-OH
Löslichkeit in Wasser: 5 Gew.-% bei Raumtemperatur.

Beispiel 8 (Natriumsalz): In einen Vierhalskolben mit mechanischem Rührer, Rückflußkühler und Thermometer werden 20.81 g (0.03 mol) der Verbindung aus Beispiel 6 und 3,6 g (0,09 mol) NaOH in 22,5 ml Wasser suspendiert. Die Supension wird 3 h bei 80°C gerührt und auf Raumtemperatur abgekühlt. Man erhalt eine orangerote, klare, leicht viskose Lösung des Salzes.
Produkt der Formel Ia (R₃ = CH₃, R₁ = CO₂Na)

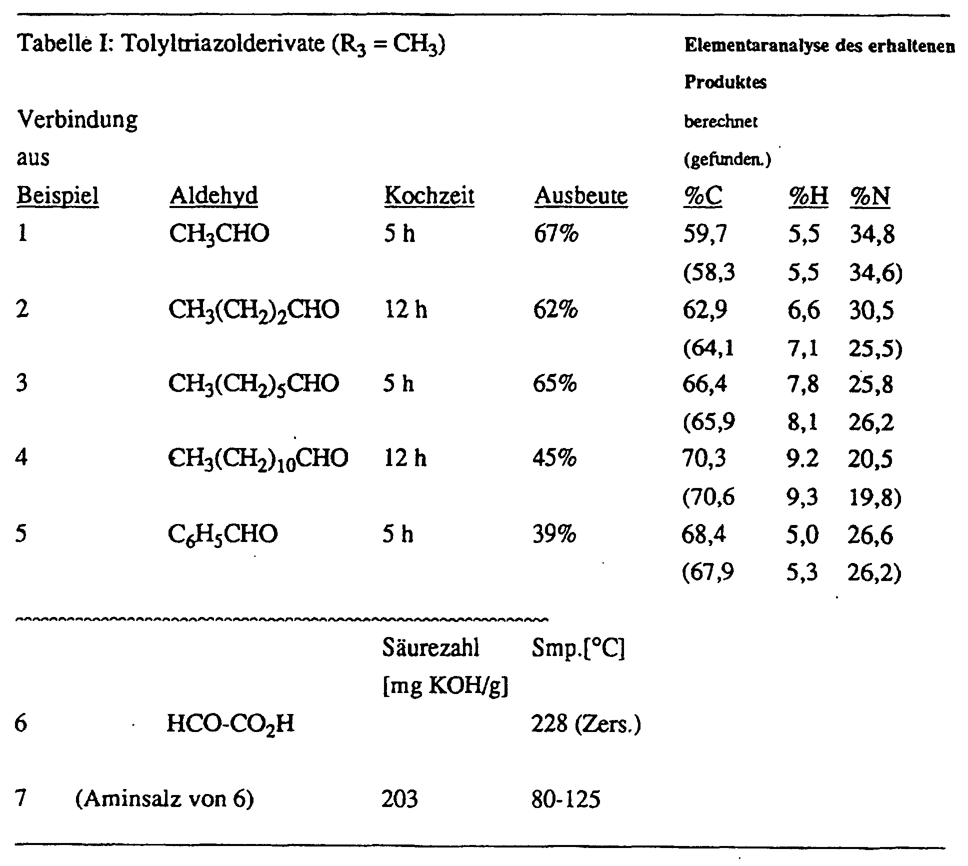

Beispiele 9 und 14: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 17,87 g (0,15 mol) Benzotriazol, 0,15 mol Paracetaldehyd bzw. Paraformaldehyd und 0,2 g para-Toluolsulfonsäure in 100 ml Toluol suspendiert. Die Mischung wird 5 Stunden unter Rückfluß gekocht und währenddessen das entstehenden Wasser azeotrop abdestilliert. Das Produkt wird abfiltriert und bei 80° unter vermindertem Druck getrocknet. Man erhält jeweils gelborangefarbene Feststoffe.

Beispiel 9: Produkt der Formel Ia (R₁ = CH₃, R₃ = H), Smp. 95°C

Beispiel 14: Produkt der Formel Ia (R₁ = H, R₃ = H), Smp. 193-212°C

Beispiel 10: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 17,87 g (0,15 mol) Benzotriazol, 13,5 ml (0,15 mol) Butyraldehyd und 0,2 g para-Toluolsulfonsäure in 100 ml Toluol suspendiert. Die Mischung wird 5 Stunden unter Rückfluß gekocht, und währenddessen wird das entstehenden Wasser azeotrop abdestilliert.
Das Lösungsmittel wird abdekantiert, und der klebrige Rückstand wird in Methylenchlorid gelöst. Nach Abdestillieren des Lösungsmittels erhält man 8,87 g eines gelborangefarbenen Feststoffes:
Produkt der Formel Ia (R₁ = CH₃(CH₂)₂, R₃ = H), Smp 74-92°C

Beispiele 11, 12 und 13: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 17,87 g (0,15 mol) Benzotriazol, 0,15 mol des betreffenden Aldehyds (vgl. Tabelle II) und 0,2 g para-Toluolsulfonsäure in 100 ml Toluol suspendiert. Die Mischung wird 4 oder 5 Stunden (s. Tabelle II) unter Rückfluß gekocht, und währenddessen wird das entstehenden Wasser azeotrop abdestilliert. Die Reaktionslösung wird dreimal mit je 100 ml 5%iger Na₂CO₃-Lösung sowie zweimal mit je 100 ml Wasser gewaschen, über wasserfreiem MgSO₄ getrocknet, und das Lösungsmittel wird abdestilliert. Man erhält jeweils gelbliche Feststoffe:

Beispiel 11: Produkt der Formel Ia (R₁ = CH₃(CH₂)₅, R₃ = H), Smp. 79-89°C

Beispiel 12: Produkt der Formel Ia (R₁ = CH₃(CH₂)₁₀, R₃ = H), glasiges Harz

Beispiel 13: Produkt der Formel Ia (R₁ = C₆H₅, R₃ = H), Smp. 130-142°C

Beispiel 15: In einem 4-Hals-Kolben mit mechanischem Rührer, Rückflußkühler, Thermometer und Stickstoffeinleitung werden unter Stickstoff 12,62 g (0,1 mol) Melamin, 27.62 g (0,3 mol) Glyoxylsäure und 35,74 g (0,3 mol) Benzotriazol in 150 ml Ethanol und 150 ml Wasser suspendiert. Die Mischung wird 5 Stunden unter Rückfluß gekocht und auf Raumtemperatur abgekühlt. Das Produkt wird abfiltriert, mit 200 ml Wasser gewaschen und im Vakuumtrockenschrank bei 70°C getrocknet. Man erhält 16.9 g (26% d.Th.) eines weißlichen Feststoffes.
Produkt der Formel Ia (R₃ = H, R₁ = CO₂H)

Beispiel 16: 6.51 g (0,01 mol) der Verbindung aus Beispiel 15 und 2,68 g (0.03 mol) 2-Amino-2-methyl-1-propanol werden in 100 ml Wasser gelöst und eine Stunde bei Raumtemperatur gerührt. Das Wasser wird am Rotationsverdampfer abdestilliert, und man erhält 7,4 g (81% d.Th.) eines gelblichen, glasigen Feststoffes.
Produkt der Formel Ia (R₃ = H, R₁ = CO₂^{⊖ ⊕}H₃N-C(CH₃)₂-CH₂-OH)
Löslichkeit in Wasser 5 Gew.-% bei Raumtemperatur.

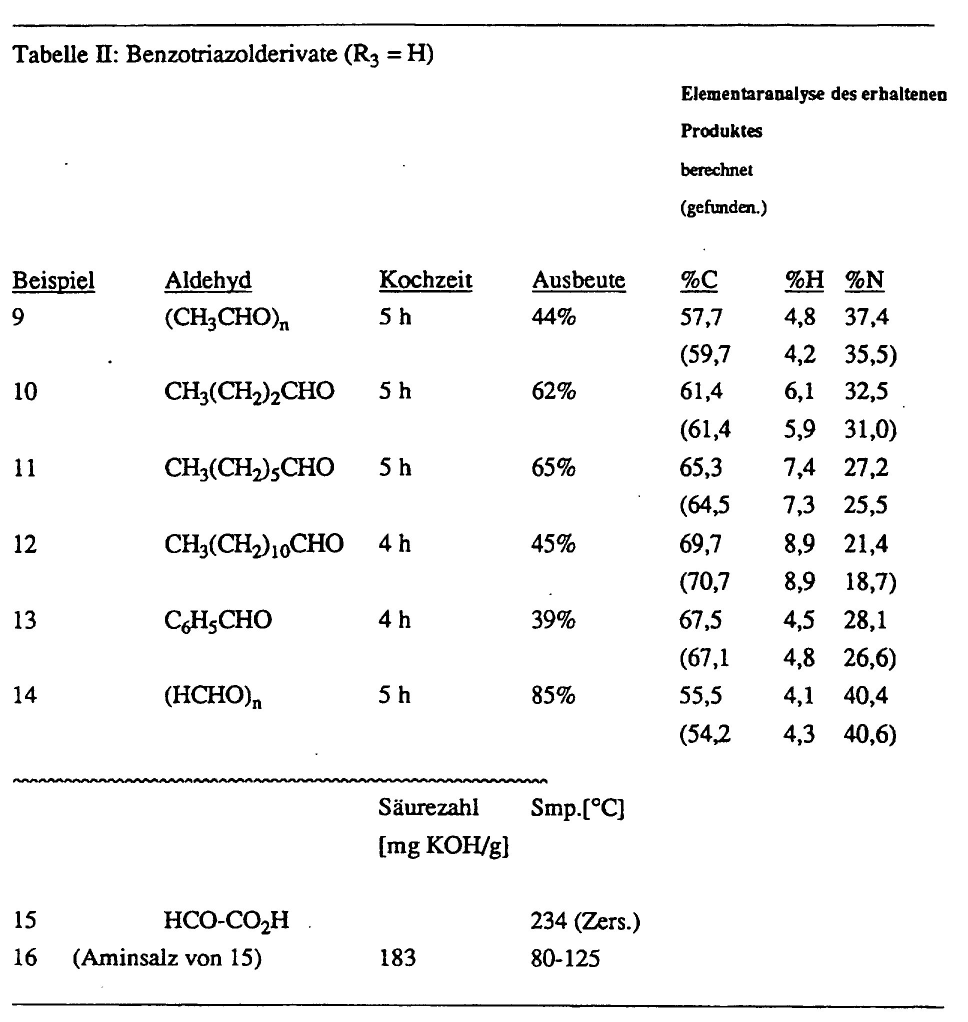

### Beispiele 17 bis 25:

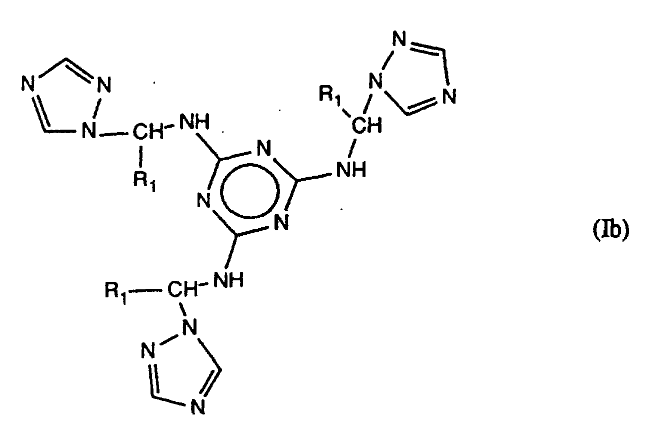

Beispiel 17: In einem 4-Hals-Kolben mit mechanischem Rührer, Rückflußkühler, Thermometer und Stickstoffeinleitung werden unter Stickstoff 6,31 g (0,05 mol) Melamin, 10,35 g (0,15 mol) 1,2,4-Triazol und 12,5g (0,15 mol) Formaldehyd (36 %ige wäßrige Lösung) in 300 ml Methanol suspendiert. Die Mischung wird 12 Stunden unter Rückfluß gekocht und zur Entfernung von nicht umgesetztem Edukt filtriert. Das Filtrat wird eingedampft und der weiße Rückstand im Vakuum (0,1 Torr, 80°C) getrocknet. Man erhält das Produkt mit 70,3 % Ausbeute: Produkt der Formel Ib (R₁ = H).

Beispiel 18: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 10,35 g (0,15 mol) 1,2,4-Triazol, 4,5 g (0,15 mol) Paraformaldehyd und 0,1 g para-Toluolsulfonsäure in 100 ml Toluol suspendiert. Die Mischung wird 2 Stunden unter Rückfluß gekocht und währenddessen das entstehenden Wasser azeotrop abdestilliert. Das Produkt wird abfilitriert und aus Wasser umkristallisiert. Nach Trocknen im Vakuum (0,1 Torr, 150°C) erhält man mit einer Ausbeute von 43% d.Th.
einen weißen Feststoff: Produkt der Formel Ib (R₁ = H).

Beispiel 19: In einem 4-Hals-Kolben mit mechanischem Rührer, Rückflußkühler, Thermometer und Stickstoffeinleitung werden unter Stickstoff 12,62 g (0,1 mol) Melamin, 20.72 g (0,3 mol) 1,2,4-Triazol und 27,5 ml (0,5 mol) Acetaldehyd in 300 ml Methanol suspendiert. Die Mischung wird 20 Stunden unter Rückfluß gekocht und am Rotationsverdampfer eingedampft. Der rosa Rückstand wird 3 Tage lang bei 60°C im Vakuumschrank getrocknet.
Ausbeute 94% d.Th.
Produkt der Formel Ib (R₁ = CH₃).

Beispiele 20 bis 23: In einem 4-Hals-Kolben mit mechanischem Rührer, Wasserabscheider nach Dean-Stark, Thermometer und Stickstoffeinleitung werden unter Stickstoff 6,31 g (0,05 mol) Melamin, 10,35 g (0,15 mol) 1,2,4-Triazol, 0,15 mol des betreffenden Aldehyds (vgl. Tabelle III) und 0,1 g para-Toluolsulfonsäure in 100 ml Toluol suspendiert. Die Mischung wird solange wie in Tabelle III angegeben unter Rückfluß gekocht und währenddessen das entstehenden Wasser azeotrop abdestilliert. Das Lösungsmittel wird abdestilliert und das jeweilige Produkt im Vakuum (0,1 Torr, 80°C) getrocknet. Man erhält jeweils gelbliche Feststoffe:

Beispiel 20: Produkt der Formel Ib (R₁ = CH₃(CH₂)₂).

Beispiel 21: Produkt der Formel Ib (R₁ = CH₃(CH₂)₁₀).

Beispiel 22: Produkt der Formel Ib (R₁ = C₆H₅).

Beispiel 23: Produkt der Formel Ib (R₁ = CH₃(CH₂)₅).

Beispiel 24: In einem 4-Hals-Kolben mit mechanischem Rührer, Rückflußkühler, Thermometer und Stickstoffeinleitung werden unter Stickstoff 12,61 g (0,1 mol) Melamin, 27.62 g (0,3 mol) Glyoxylsäure Monohydrat und 20.7 g (0,3 mol) 1,2,4-Triazol in 150 ml Ethanol und 150 ml Wasser suspendiert Die Mischung wird 22 Stunden unter Rückfluß gekocht und auf Raumtemperatur abgekühlt. Zur erhaltenen Suspension werden 500 ml Methylenchlorid gegeben, 2 h gerührt, und das Produkt wird abfiltriert. Das Produkt wird mit 50 ml Methylenchlorid gewaschen, abfiltriert und im Vakuumtrockenschrank bei 60°C getrocknet. Man erhält 19.3 g (33% d.Th.) eines gelblichen Feststoffes.
Produkt der Formel Ib (R₁ = CO₂H).

Beispiel 25: 5.02g (0,01 mol) der Verbindung aus Beispiel 24 und 2,68 g (0.03 mol) 2-Amino-2-methyl-1-propanol werden in 100 ml Wasser gelöst und 2 h bei Raumtemperatur gerührt. Das Wasser wird am Rotationsverdampfer abdestilliert, und man erhält 6,1 g (81% d.Th.) eines gelben, glasigen Feststoffes.

Produkt der Formel Ib (R₁ = COO^{⊖ ⊕}H₃N-C(CH₃)₂-CH₂-OH).
Löslichkeit in Wasser: 5 Gew.-% bei Raumtemperatur.

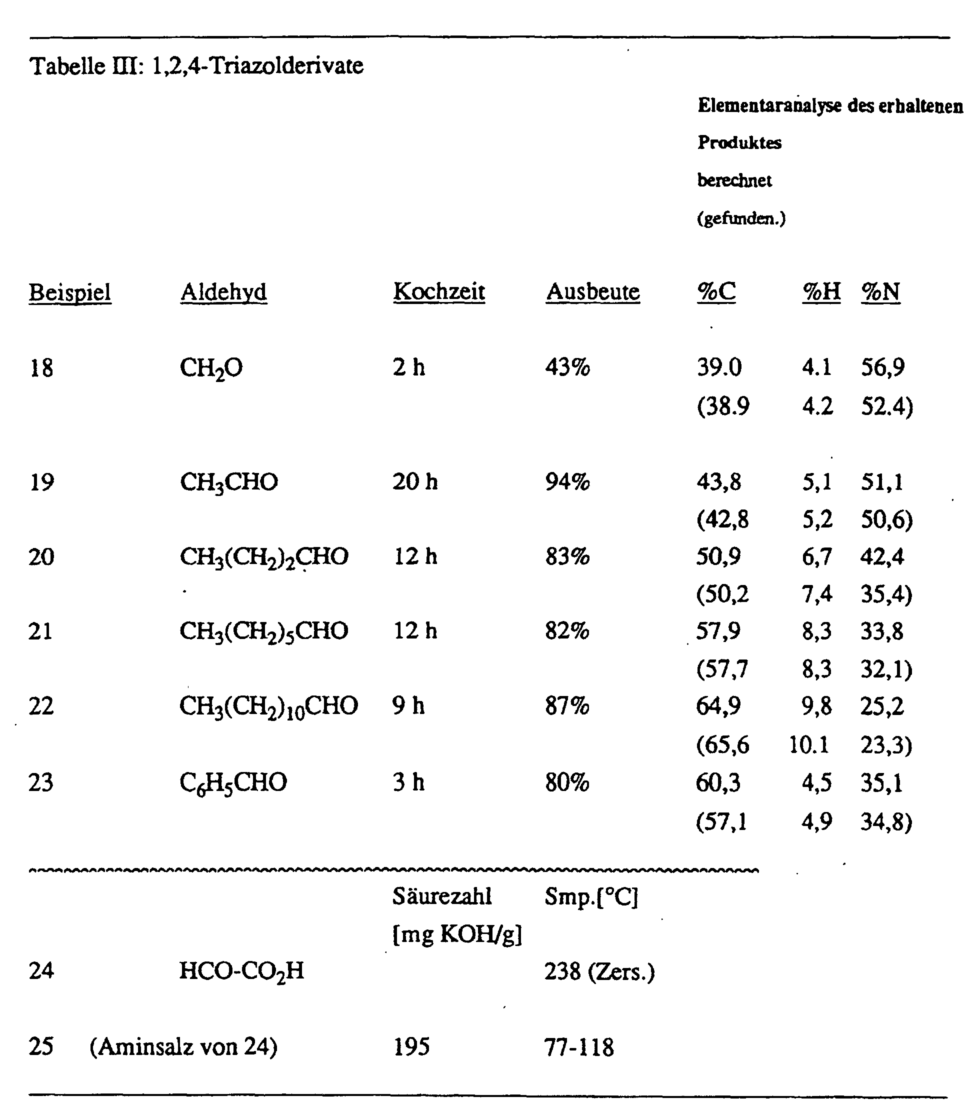

### Anwendungsbeispiele

### Beispiel A1: Kupfer Korrosionstest (modifiziert nach ASTM D-130)

0,05 Gewichts% der zu testenden Verbindung werden in einem Turbinenöl der Viskosität 29,7 mm²s⁻¹ bei 40°C und 5,05 mm² s⁻¹ bei 100°C (Schwefelgehalt 0,22%) gelöst. Weitere 50 ppm elementarer Schwefel werden zugegeben.

Ein mit Siliciumcarbid poliertes Kupferblech (60 x 10 x 1 mm) wird vollständig in die Öllösung getaucht und 3 Stunden bei 100°C dort belassen. Danach wird das Kupferblech aus dem Öl genommen und mit Petrolether gespült. Es folgt die Bewertung nach der ASTM D 130 Copper Strip Corrosion Standard Chart (Vgl. Tabelle IV). Die Beurteilung geschieht in vier Stufen:
1 - kein Beschlag
2 - mäßiger Beschlag
3 - starker Beschlag
4 - Korrosion,
wobei innerhalb der Zahlengruppen 1 bis 4 noch auf Grund der Schattenbildung auf den Proben eine Feinunterteilung vorgenommen wird. In der qualitativen Beurteilung A bis E steht dabei die Wertung A vor B, B vor C usw. Die Tabelle zeigt jeweils Werte zweier Bleche (Parallelbestimmung).

**Tabelle IV:**

| Kupfer Korrosionstest | |
|---|---|
| Verbindung aus Beispiel Nr. | Beurteilung |
| -- | 3B/4A |
| 2 | 1A/1A |
| 3 | 1A/1A |
| 4 | 1A/1B |
| 12 | 1A/1A |
| 22 | 1A/1A |

### Beispiel A2: Rotary Bomb Oxidation Test (RBOT), ASTM D 2272

0,05 Gewichts% der zu testenden Verbindung werden in einem Turbinenöl (Viskosität 29,7 mm²s⁻¹ bei 40°C und 5,05 mm² s⁻¹ bei 100°C, Schwefelgehalt 0,22%) gelöst. Weitere Komponenten sind 0,1% eines phenolischen, 0,1% eines aminischen oder je 0,1% eines phenolischen und eines aminischen Antioxidans. (Vgl. Tabelle IV). 50 ml der so erhaltenen Mischung werden mit 5 ml Wasser in das Testgefäß gegeben, das als Katalysator eine Kupferspirale enthält. Das Gefäß wird mit Sauerstoff bis zu einem Druck von 620 kPa beladen, verschlossen und in einem 150°C heißen Bad rotiert. Gemessen wird die Zeit, in welcher der Sauerstoffdruck um 172 kPa abfällt.

**Tabelle V:**

| Rotary Bomb Oxidation Test (RBOT) | | | |
|---|---|---|---|
| Verbindung aus Beisp. Nr. | Antioxidans A¹⁾ | Antioxidans B²⁾ | Zeit [min] |
| -- | -- | -- | 34 |
| -- | 0,1% | -- | 143 |
| -- | -- | 0,1% | 80 |
| | | | |
| 4 | 0,1% | -- | 447 |
| 4 | -- | 0,1% | 336 |
| 4 | 0,1% | 0,1% | 597 |
| | | | |
| 12 | 0,1% | -- | 479 |
| 12 | -- | 0,1% | 392 |

| | | | |
|---|---|---|---|
| 2) Gemisch von Diphenylamin-Verbindungen, kommerziell erhältlich als Irganox® L-57, vgl. US-5,073,278, Sp. 2, Z. 50 | | | |
| 1) Gemisch von tert-butylierten Phenolen, erhältlich als Irganox® L 140. | | | |

### Beispiel A3: Kupferkorrosion in Wasser

0,05 Gewichts% der zu testenden Verbindung werden in 75 ml hartem Wasser (DIN 51360) gelöst. Ein mit Siliciumcarbid poliertes und gewogenes Kupferblech (50 x 20 x 0,2 mm) wird vollständig in die Lösung getaucht und gut verschlossen 24 Stunden bei 60°C gelagert. Dann wird das Blech entnommen, 15 sec in 5 N Salzsäurelösung getaucht und mit deionisertem Wasser und Aceton gespült. Nach dem Trocknen wird das Kupferblech wieder gewogen.

Der Gewichtsverlust dient der Beurteilung des Korrosionsschutzes. Hoher Gewichtsverlust bedeutet starke Korrosion, geringer Gewichtsverlust guten Korrosionschutz. Tabelle VI zeigt die Werte einer Bestimmung:

| Tabelle VI: Kupfer Korrosionstest in Wasser | |
|---|---|
| Verbindung aus Beispiel Nr. | Gewichtsverlust [mg] |
| -- | 6,6 |
| 19 | 1,4 |
| 7 | 0.6 |
| 16 | 0,4 |
| 25 | 1,0 |

### Beispiel A4: Test auf Eignung als Hochdruck- und Verschleißschutzmittel

Zur Prüfung auf Eignung als Verschleißschutzadditiv wird die ASTM-Standardmethode D-2783-81 unter Verwendung des Shell-Vierkugelapparates herangezogen. Als Basisöl wird der Typ 150 SSU der Fa. Mobil verwendet, dem die in der Tabelle angegebene Menge an Verbindung gemäß dem jeweils genannten Beispiel zugegeben wird. Ermittelt wird der mittlere Verschleiß-Narben-Durchmesser WSD (Wear-Scar-Diameter) bei einer Last von 400 N nach einer Stunde Betrieb bei 25°C und 1440 UpM (in mm).

Die erhaltenen Resultate sind in Tabelle VII aufgeführt. Geringer Durchmesser der Verschleißnarbe bedeutet Eignung als Verschleißschutzmittel.

**Tabelle VII:**

| Verbindung aus Beispiel | Zusatzmenge [%] | WSD [mm] |
|---|---|---|
| ohne Zusatz | - | 0,93 |
| 4 | 0,25 | 0,64 |
| | 1,0 | 0,60 |

## Patentansprüche

1. Verbindungen der Formel I 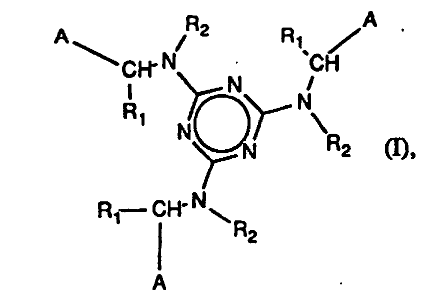 worin
A für 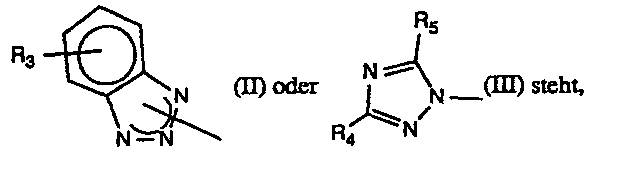 R₁ Wasserstoff, C₁-C₂₀-Alkyl, C₂-C₂₀-Alkenyl, C₅-C₁₂-Cycloalkyl, mit C₁-C₁₀-Alkyl substituiertes C₅-C₁₂-Cycloalkyl, Phenyl, Naphthyl, mit C₁-C₁₀-Alkyl, C₁-C₁₀-Alkoxy, Halogen, Hydroxy, Nitro oder Phenoxy substituiertes Phenyl oder Naphthyl, eine Gruppe der Formel 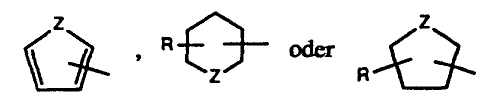 ist, wobei R Wasserstoff oder C₁-C₁₀-Alkyl ist und
Z für Sauerstoff, -Schwefel, -NH-, -NR₆- oder eine Methylengruppe steht,
oder R₁ COOR₇ darstellt,
R₂ Wasserstoff, C₁-C₂₀-Alkyl oder C₅-C₁₂-Cycloalkyl ist,
R₃ Wasserstoff, C₁-C₅-Alkyl, C₁-C₅-Alkoxy, Hydroxy, Halogen, Nitro, Carboxy oder
C₂-C₁₁-Alkoxycarbonyl ist,
R₄ und R₅ unabhängig voneinander -SH, -NH₂, -NHR₆, -NO₂, -COOH, -SR₆, -N(R₆)₂, -COO-R₆ oder Maleinimido sind,
R₆ C₁-C₁₀Alkyl bedeutet,
R₇ für Wasserstoff, Mⁿ⁺/n oder [NR₈R₉R₁₀R₁₁]⁺ steht,
M Alkali oder Erdalkalimetall bedeutet, n 1 oder 2 ist, und
R₈ bis R₁₁ unabhängig voneinander Wasserstoff, C₁-C₈-Alkyl oder C₁-C₈-Hydroxyalkyl sind.

2. Verbindungen der Formel I, worin
R₁ Wasserstoff, C₁-C₁₈-Alkyl, C₂-C₁₈-Alkenyl, Phenyl, Cyclohexyl, mit C₁-C₄-Alkyl substituiertes Cyclohexyl oder COOR₇ ist,
R₂ Wasserstoff, C₁-C₁₂-Alkyl oder Cyclohexyl ist,
R₃ Wasserstoff, Chlor, C₁-C₅-Alkyl, Methoxy, Hydroxy oder Nitro ist,
R₄ und R₅ unabhängig voneinander für Wasserstoff, SH, NH₂ oder NO₂ stehen.

3. Verbindungen nach Anspruch 1, worin
R₁ C₁-C₁₂-Alkyl, C₂-C₁₂-Alkenyl, COOR₇ oder Phenyl ist.

4. Verbindungen nach Anspruch 1, worin
R₂ Wasserstoff oder C₁-C₄-Alkyl ist.

5. Verbindungen nach Anspruch (2), worin
R₁ C₁-C₁₂-Alkyl, Phenyl, COOH, oder COOR₇ ist, und
R₂, R₃, R₄ und R₅ Wasserstoff oder C₁-C₄-Alkyl sind,

6. Verbindungen nach Anspruch 1, worin
R₃ Wasserstoff, C₁-C₅-Alkyl, C₁-C₅-Alkoxy oder Hydroxy ist.

7. Verbindungen nach Anspruch 1, worin
R₃ Wasserstoff, Methyl oder Butyl ist.

8. Die Verbindung der Formel I nach Anspruch 1, worin R₁ n-C₁₁H₂₃, R₂ H, A ein Rest II und R₃ H sind, die Verbindung der Formel I nach Anspruch I, worin R₁ -COOH R₂ H, A ein Rest II und R₃ CH₃ sind, die Verbindung der Formel I nach Anspruch I, worin R₁ COOH, A ein Rest II, R₂ H und R₃ H sind, sowie die Verbindung der Formel I nach Anspruch I, worin R₁ CH₃, A ein Rest III und R₂, R₄ und R₅ H sind.

9. Verbindungen nach Anspruch 1 der Formel **Ia** 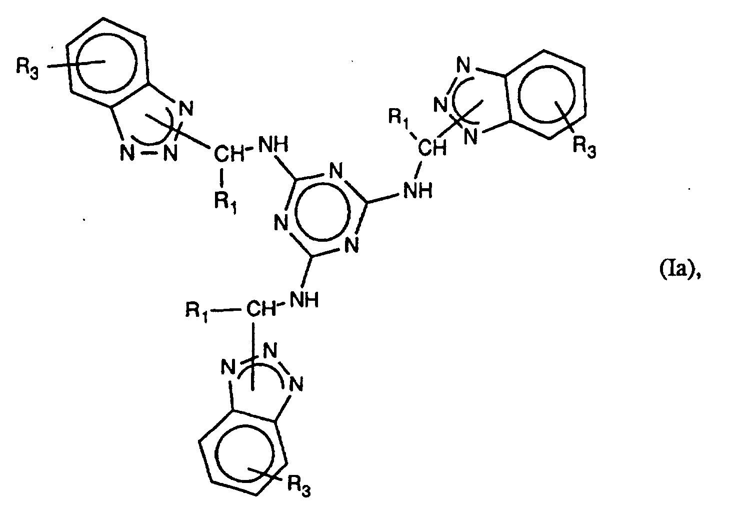 worin R₁ Wasserstoff, C₁-C₂₀-Alkyl, C₂-C₂₀-Alkenyl, C₅-C₁₂-Cycloalkyl, mit C₁-C₁₀-Alkyl substituiertes C₅-C₁₂-Cycloalkyl, Phenyl, Naphthyl, mit C₁-C₁₀-Alkyl, C₁-C₁₀-Alkoxy, Halogen, Hydroxy, Nitro oder Phenoxy substituiertes Phenyl oder Naphthyl, eine Gruppe der Formel 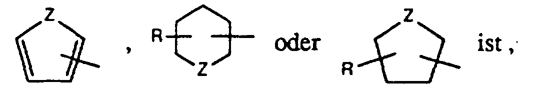 wobei R für Wasserstoff oder C₁-C₁₀-Alkyl steht, Z Sauerstoff oder Schwefel ist, und R₃ Wasserstoff, C₁-C₅-Alkyl, C₁-C₅-Alkoxy, Hydroxy, Halogen, Nitro, Carboxy oder C₂-C₁₁-Alkoxycarbonyl ist.

10. Verbindungen nach Anspruch **9**, worin
R₃ Wasserstoff, C₁-C₅-Alkyl, C₁-C₅-Alkoxy oder Hydroxy ist.

11. Verbindungen nach Anspruch **9,** worin
R₁ C₁-C₁₂-Alkyl, C₂-C₁₂-Alkenyl oder Phenyl ist.

12. Zusammensetzungen enthaltend
A) einen Schmierstoff, eine Hydraulik- oder Metallbearbeitungsflüssigkeit oder ein Beschichtungs- oder Überzugsmittel und
B) mindestens eine Verbindung der Formel I nach Anspruch 1.

13. Zusammensetzungen nach Anspruch **12**, worin die Komponente A) ein Schmierstoff ist.

14. Zusammensetzungen nach Anspruch **13,** worin der Schmierstoff ein Motoröl ist.

15. Zusammensetzungen nach Anspruch **12,** die zusätzlich weitere Stabilisatoren wie weitere Antioxidantien, weitere Metalldesaktivatoren, Rostinhibitoren, Viskositäts-Index-Verbesserer, Stockpunkterniedriger, Dispergiermittel/Tenside und/oder Hochdruck- und Verschleißschutzadditive enthalten.

16. Zusammensetzungen nach Anspruch **15**, die als weitere Antioxidantien phenolische und/oder aminische Antioxidantien enthalten.

17. Verwendung von Verbindungen der Formel I gemäß Anspruch **1** als Additive in Schmierstoffen, Hydraulik- und Metallbearbeitungsflüssigkeiten oder Beschichtungs- und Überzugsmitteln.

## Claims

1. A compound of formula I 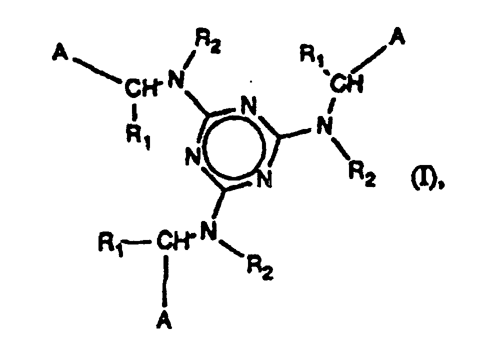 wherein
A is 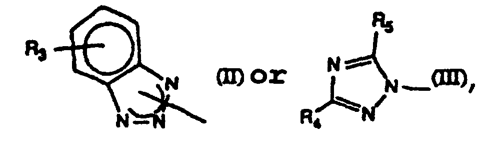 R₁ is hydrogen, C₁-C₂₀alkyl, C₂-C₂₀alkenyl, C₅-C₁₂-cycloalkyl, or C₅-C₁₂cycloalkyl which is substituted by C₁-C₁₀alkyl; phenyl, naphthyl,; phenyl or naphthyl, each of which is substituted by C₁-C₁₀alkyl, C₁-C₁₀alkoxy, halogen, hydroxy, nitro or phenoxy; a group of formula 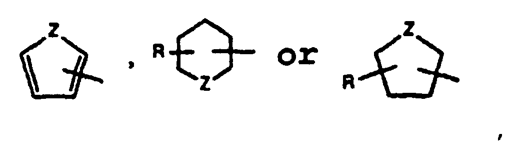 in which R is hydrogen or
C₁-C₁₀alkyl and
Z is oxygen, sulfur, -NH-, -NR₆- or a methylene group,
or R₁ is COOR₇,
R₂ is hydrogen, C₁-C₂₀alkyl or C₅-C₁₂cycloalkyl,
R₃ is hydrogen, C₁-C₅alkyl, C₁-C₅alkoxy, hydroxy, halogen, nitro, carboxy or C₂-C₁₁alkoxycarbonyl,
R₄ and R₅ are each independently of the other -SH, -NH₂, -NHR₆, -NO₂, -COOH, -SR₆, -N(R₆)₂, -COO-R₆ or maleimido,
R₆ is C₁-C₁₀alkyl,
R₇ is hydrogen, Mⁿ⁺/n or [NR₈R₉R₁₀R₁₁]⁺,
M is alkali or alkaline earth metal, n is 1 or 2, and
R₈ to R₁₁ are each independently of one another hydrogen, C₁-C₈alkyl or C₁-C₈hydroxyalkyl.

2. A compound of formula I wherein
R₁ is hydrogen, C₁-C₁₈alkyl, C₂-C₁₈alkenyl, phenyl, cyclohexyl, C₁-C₄alkyl-substituted cyclohexyl, or COOR₇,
R₂ is hydrogen, C₁-C₁₂alkyl or cyclohexyl,
R₃ is hydrogen, chloro, C₁-C₅alkyl, methoxy, hydroxy or nitro,
R₄ and R₅ are each independently of the other hydrogen, SH, NH₂ or NO₂.

3. A compound according to claim 1, wherein R₁ is C₁-C₁₂alkyl, C₂-C₁₂alkenyl, COOR₇ or phenyl.

4. A compound according to claim 1, wherein R₂ is hydrogen or C₁-C₄alkyl.

5. A compound according to claim 2, wherein R₁ is C₁-C₁₂alkyl, phenyl, COOH, or COOR₇, and R₂, R₃, R₄ and R₅ are hydrogen or C₁-C₄alkyl.

6. A compound according to claim 1, wherein R₃ is hydrogen, C₁-C₅alkyl, C₁-C₅alkoxy or hydroxy.

7. A compound according to claim 1, wherein R₃ is hydrogen, methyl or butyl.

8. A compound of formula I according to claim 1, wherein R₁ is n-C₁₁H₂₃, R₂ is H, A is a radical II, and R₃ is H; a compound of formula I according to claim 1, wherein R₁ is -COOH, R₂ is H, A is a radical II, and R₃ is CH₃; a compound of formula I according to claim 1, wherein R₁ is COOH, A is a radical II, R₂ is H, and R₃ is H; and a compound of formula I according to claim 1, wherein R₁ is CH₃, A is a radical III, and R₂, R₄ and R₅ are H.

9. A compound according to claim 1, of formula Ia 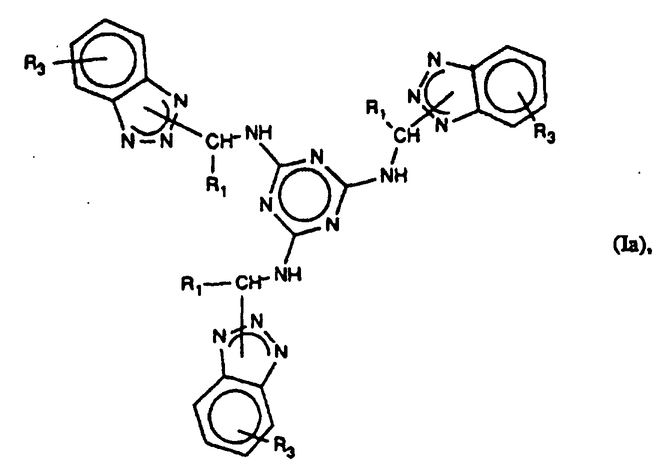 wherein R₁ is hydrogen, C₁-C₂₀alkyl, C₂-C₂₀alkenyl, C₅-C₁₂cycloalkyl, or C₅-C₁₂cycloalkyl which is substituted by C₁-C₁₀alkyl; phenyl, naphthyl; phenyl or naphthyl, each of which is substituted by C₁-C₁₀alkyl, C₁-C₁₀alkoxy, halogen, hydroxy, nitro or phenoxy; a group of formula 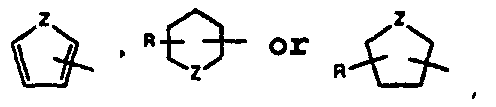 in which R is hydrogen or C₁-C₁₀alkyl, Z is oxygen or sulfur, and
R₃ is hydrogen, C₁-C₅alkyl, C₁-C₅alkoxy, hydroxy, halogen, nitro, carboxy or C₂-C₁₁alkoxycarbonyl.

10. A compound according to claim **9**, wherein R₃ is hydrogen, C₁-C₅alkyl, C₁-C₅alkoxy or hydroxy.

11. A compound according to claim **9**, wherein R₁ is C₁-C₁₂alkyl, C₂-C₁₂alkenyl or phenyl.

12. A composition comprising
A) a lubricant, a hydraulic fluid or a metalworking fluid, or a coating composition, and
B) at least one compound of formula I according to claim 1.

13. A composition according to claim **12**, wherein component A) is a lubricant.

14. A composition according to claim **13**, wherein the lubricant is a motor oil.

15. A composition according to claim **12**, additionally comprising further stabilizers such as further antioxidants, further metal deactivators, rust inhibitors, viscosity index improvers, pour-point depressants, dispersants/surfactants and/or highpressure and antiwear additives.

16. A composition according to claim **15**, comprising as further antioxidants phenolic and/or aminic antioxidants.

17. The use of compounds of formula I according to claim **1** as additives in lubricants, hydraulic fluids and metalworking fluids, or coating compositions.

## Revendications

1. Composé de formule I où 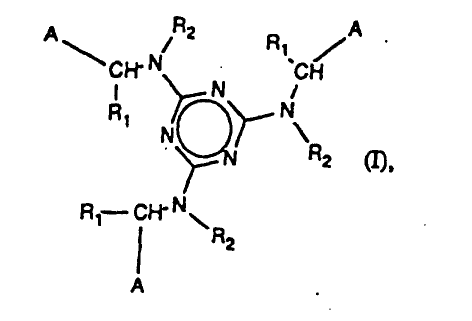 A représente 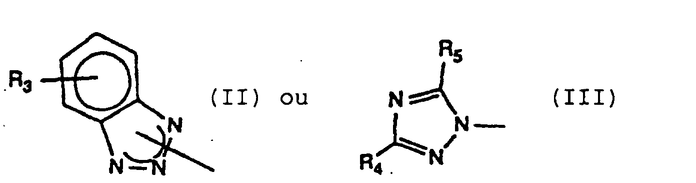 R₁ représente un atome d'hydrogène, des groupes alkyle en C₁-C₂₀, alcényle en C₂-C₂₀, cycloalkyle en C₅-C₁₂ ou cycloalkyle en C₅-C₁₂ substitué par un substituant alkyle en C₁-C₁₀, phényle, naphtyle, phényle ou naphtyle substitué par des substituants alkyle en C₁-C₁₀, alkoxy en C₁-C₁₀, halogène, hydroxy, nitro ou phénoxy, un groupe de formule 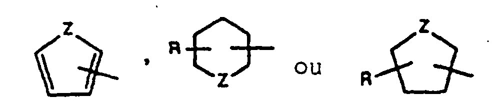 R représentant un atome d'hydrogène ou un groupe alkyle en C₁-C₁₀, et
Z représente un atome d'oxygène, un atome de soufre, des groupes -NH-, -NR₆- ou méthylène,
ou
R₁ représente un groupe COOR₇,
R₂ représente un atome d'hydrogène, des groupes alkyle en C₁-C₂₀ ou cycloalkyle en C₅-C₁₂,
R₃ représente un atome d'hydrogène, des groupes alkyle en C₁-C₅, alkoxy en C₁-C₅, hydroxy, halogène, nitro, carboxy ou (alkoxy en C₂-C₁₁)carbonyle,
R₄ et R₅ représentent, indépendamment l'un de l'autre, des groupes -SH, -NH₂, -NHR₆, -NO₂, -COOH, -SR₆, -N(R₆)₂, -COO-R₆ ou maléimido,
R₆ représente un groupe alkyle en C₁-C₁₀,
R₇ représente un atome d'hydrogène, des groupes Mⁿ⁺/n ou [NR₈R₉R₁₀R₁₁]^{*+*},
M représente un métal alcalin ou alcalino-terreux, n vaut 1 ou 2, et
R₈ à R₁₁ représentent, indépendamment les uns des autres, des atomes d'hydrogène, des groupes alkyle en C₁-C₈ ou hydroxyalkyle en C₁-C₈.

2. Composés de formule I, où
R₁ représente un atome d'hydrogène, des groupes alkyle en C₁-C₁₈, alcényle en C₂-C₁₈, phényle, cyclohexyle, cyclohexyle substitué par un substituant alkyle en C₁-C₄ ou COOR₇,
R₂ représente un atome d'hydrogène, des groupes alkyle en C₁-C₁₂ ou cyclohexyle,
R₃ représente un atome d'hydrogène, un atome de chlore, des groupes alkyle en C₁-C₅, méthoxy, hydroxy ou nitro, et
R₄ et R₅ représentent, indépendamment l'un de l'autre, des atomes d'hydrogène, des groupes SH, NH₂ ou NO₂.

3. Composés selon la revendication I, où R₁ représente des groupes alkyle en C₁-C₁₂, alcényle en C₂-C₁₂, COOR₇ ou phényle.

4. Composés selon la revendication 1, où R₂ représente un atome d'hydrogène ou un groupe alkyle en C₁-C₄.

5. Composés selon la revendication 2, où R₁ représente des groupes alkyle en C₁-C₁₂, phényle, COOH ou COOR₇ et
R₂, R₃, R₄ et R₅ représentent un atome d'hydrogène, un groupe alkyle en C₁-C₄.

6. Composés selon la revendication 1, où R₃ représente un atome d'hydrogène, des groupes alkyle en C₁-C₅, alkoxy en C₁-C₅ ou hydroxy.

7. Composés selon la revendication 1, où R₃ représente un atome d'hydrogène, des groupes méthyle ou butyle.

8. Composés de formule I selon la revendication 1, où R₁ représente un groupe n-C₁₁H₂₃, R₂ un atome d'hydrogène, A un reste II et R₃ un atome d'hydrogène, les composés de formule I selon la revendication 1, où R₁ représente un groupe COOH, R₂ un atome d'hydrogène, A un reste de formule II et R₃ un groupe CH₃, les composés de formule I selon la revendication 1, où R₁ représente un groupe COOH, A un reste II, R₂ un atome d'hydrogène et R₃ un atome d'hydrogène, ainsi que des composés de formule I selon la revendication 1, où R₁ représente un groupe CH₃, A un reste III et R₂, R₄ et R₅ représentent un atome d'hydrogène.

9. Composés selon la revendication 1 de formule Ia 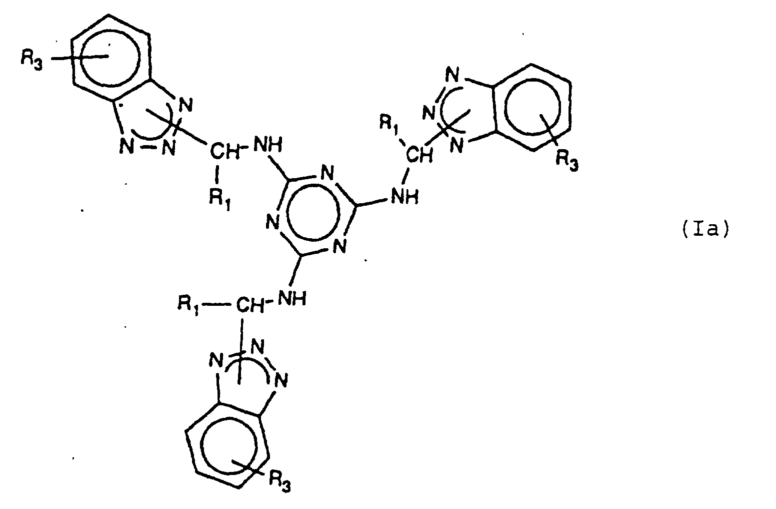 où
R₁ représente un atome d'hydrogène, des groupes alkyle en C₁-C₂₀, alcényle en C₂-C₂₀, cycloalkyle en C₅-C₁₂, cycloalkyle en C₅-C₁₂ substitués par un substituant alkyle en C₁-C₁₀, phényle, naphtyle, phényle ou naphtyle substitué par des substituants alkyle en C₁-C₁₀, alkoxy en C₁-C₁₀, halogène, hydroxy, nitro ou phénoxy, un groupe de formule 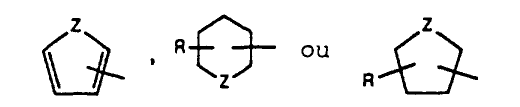 R représentant un atome d'hydrogène ou un groupe alkyle en C₁-C₁₀, et
Z représente un atome d'oxygène ou un atome de soufre, et
R₃ représente un atome d'hydrogène, des groupes alkyle en C₁-C₅, alkoxy en C₁-C₅, hydroxy, halogène, nitro, carboxy ou (alkoxy en C₂-C₁₁)carbonyle.

10. Composés selon la revendication 9, où R₃ représente un atome d'hydrogène, des groupes alkyle en C₁-C₅, alkoxy en C₁-C₅ ou hydroxy.

11. Composés selon la revendication 9, où R₁ représente des groupes alkyle en C₁-C₁₂, alcényle en C₂-C₁₂ ou phényle.

12. Composition contenant
A) un lubrifiant, un fluide hydraulique ou un fluide pour le travail de métaux ou un agent d'enduction ou de revêtement et
B) au moins un composé de formule I selon la revendication 1.

13. Compositions selon la revendication 12, où le composant A est un lubrifiant.

14. Compositions selon la revendication 13, où le lubrifiant et une huile de moteur.

15. Compositions selon la revendication 12, contenant de plus d'autres stabilisants tels que d'autres antioxydants, d'autres désactivateurs de métaux, inhibiteurs de rouille, agents améliorant l'indice de viscosité, agent abaissant le point de figeage, agents dispersants/agents de surface et/ou additifs haute pression/anti-usure.

16. Compositions selon la revendication 15, contenant comme d'autres antioxydants des antioxydants phénoliques et/ou aminés.

17. Utilisation de composés de formule I selon la revendication 1 comme additifs dans des lubrifiants, des fluides hydrauliques et des fluides pour le travail de métaux ou des agents d'enduction et de revêtement.
